# EUROPÄISCHE PATENTSCHRIFT

(11) **EP 0 932 640 B1**
(45) Veröffentlichungstag und Bekanntmachung des Hinweises auf die Patenterteilung: **26.04.2000**
(21) Anmeldenummer: 97943653.2
(22) Anmeldetag: 09.10.1997
(51) Int. Cl.: C08J 5/18, A61C 15/04

(54) **FIBRILLATIONSARMER FORMKÖRPER**
LOW-FIBRILLATION MOULDED BODY
CORPS FACONNE FAIBLEMENT FIBRILLE

(30) Priorität: 11.10.1996 AT 179896
(43) Veröffentlichungstag der Anmeldung: 04.08.1999
(73) Patentinhaber: LENZING AKTIENGESELLSCHAFT, 4860 Lenzing (AT)
(72) Erfinder: WIMMER, Adalbert, A-4840 Vöcklabruck (AT)
(74) Vertreter: Nemec, Harald, Dipl.-Ing.
(86) Internationale Anmeldenummer: AT9700215
(87) Internationale Veröffentlichungsnummer: WO9816572

(56) Entgegenhaltungen:
- EP-A- 0 172 671
- EP-A- 0 358 363
- WO-A-93/02633
- WO-A-95/34252

## Beschreibung

Die Erfindung betrifft ein Verfahren zur Herstellung eines fibrillationsarmen Formkörpers enthaltend Polytetrafluorethylen, fibrillationsarme Formkörper, welche durch das erfindungsgemäße Verfahren erhältlich sind sowie deren Verwendung als Zahnseide material.

Polytetrafluorethylen (PTFE) ist aufgrund seiner thermischen Stabilität und seiner chemischen Inertheit ein geschätzter Werkstoff. Aus der AT-B 370.673 sind monoaxial verstreckte Folien aus gesintertem PTFE bekannt, deren Festigkeit in Verstreckungsrichtung Werte zwischen 50 N/mm² und 140 N/mm² aufweist. Diese Folien werden hergestellt, indem PTFE-Pulver zunächst zu einem zylindrischen Formkörper verpreßt wird. Anschließend wird der Formkörper gesintert, worauf Folien abgeschält, auf Temperaturen von mindestens 327 °C erhitzt und verstreckt werden.

Die GB-A 2 025 835 beschreibt die Herstellung poröser PTFE-Formkörper nach dem Pastenextrusionsverfahren, wobei eine pastenförmige Masse, welche im wesentlichen PTFE-Pulver und ein Gleitmittel (Kohlenwasserstoff) enthält, durch Düsen gepreßt wird, worauf das Gleitmittel durch Trocknen entfernt wird. Danach wird der Formkörper über den Kristallitschmelzpunkt des PTFE (327 °C) erhitzt und während des Erhitzens verstreckt.

Die EP-A 0 391 887 beschreibt ein Verfahren zur Herstellung eines monoaxial verstreckten Formkörpers aus PTFE, bei welchem eine pastenförmige PTFE-Masse kontinuierlich zu einem Formling verarbeitet wird, welcher über eine Mehrzahl von Rollen bzw. Walzen geführt, erhitzt und verstreckt wird, wobei vor Aufbringen des Streckzuges der Formling auf eine Temperatur zwischen 327 °C und 450 °C erhitzt und dabei gesintert wird. Dieses Verfahren gestattet die Herstellung eines monoaxialen Formkörpers aus PTFE mit Festigkeitswerten in Verstreckungsrichtung von mindestens 22 cN/tex (500 N/mm²).

Es ist weiters bekannt, daß Formkörper aus PTFE mit verschiedenen Füllstoffen zur Verfügung gestellt werden können. Solche Füllstoffe enthaltende Formkörper sind z.B. aus der AT-B 399 882 bekannt und enthalten als Füllmittel Talk und/oder Glimmer und/oder ein hochtemperaturbeständiges Polyimid zwischen 20 und 30 Massen-%. Die in der AT-B 399 882 beschriebenen Füllstoffe enthaltenden PTFE-Formkörper eignen sich z.B. zur Verwendung als Zahnseide.

Zahnseiden aus PTFE-Bändchen sind außerdem z.B. in den EP-A 0 335 466, US-A 5,209,251 und US-A 5,220,932 beschrieben. Die PTFE-Bändchen können dabei unterschiedliche Dichte aufweisen und im gefalteten oder ungefalteten Zustand vorliegen.

Im Vergleich zu herkömmlichen Zahnseiden z.B. aus Polyamid ist das PTFE-Material weicher, was eine höhere Geschmeidigkeit und Weichheit und damit bei der Verwendung eine verringerte Verletzungsgefahr am Zahnfleisch mit sich bringt.

Aufgrund des gereckten oder expandierten Zustandes neigen PTFE-Bändchen jedoch zur Fibrillierung, also zur Aufspaltung einzelner Fasern. Dies bringt Probleme entweder bereits beim Abwickeln der Zahnseide aus dem Zahnseidespender bzw. wenn die Zahnseide bei der Anwendung aufspleißt und Reste zwischen Zähnen hinterläßt.

In der PCT-WO 95/34252 wird eine Zahnseide aus einer ungefalteten expandierten PTFE-Faser vorgeschlagen, welche über ihre Länge gleichförmige Dicke und Breite aufweist und wobei die parallel ausgesetzten Kanten der Zahnseide widerstandsfähig gegen Fibrillierung sind. Dabei wird ein im Vergleich zum Stand der Technik dickeres PTFE-Blatt in gleichförmig dicke und breite Stränge zerschnitten. Die Stränge werden ohne vorherige Faltung aufgerollt.

Die vorliegende Erfindung stellt sich zur Aufgabe, einen PTFE enthaltenden Formkörper, der sich insbesondere als Zahnseidematerial eignet, sowie ein Verfahren zu dessen Herstellung zur Verfügung zu stellen, wobei der Formkörper besonders widerstandsfähig gegen Fibrillierung sein soll. Das Verfahren zur Herstellung des Formkörpers soll einfach und kostengünstig durchführbar sein.

Die Aufgabe der vorliegenden Erfindung wird durch ein Verfahren zur Herstellung eines fibrillationsarmen Formkörpers enthaltend Polytetrafluorethylen gelöst, welches dadurch gekennzeichnet ist, daß ein Vorformkörper aus Polytetrafluorethylen verdreht und anschließend wiederum flachgedrückt wird.

Es hat sich überraschenderweise gezeigt, daß durch die Maßnahme, einen PTFE enthaltenden Vorformkörper zu verdrehen und den verdrehten Vorformkörper wieder flachzudrücken, Formkörper erhältlich sind, welche besonders widerstandsfähig gegen Fibrillierung sind. Das erfindungsgemäße Verfahren zeichnet sich dabei durch seine einfache Durchführbarkeit aus. Das erfindungsgemäße Verfahren kann auch kontinuierlich durchgeführt werden.

Als Vorformkörper wird für die Zwecke der vorliegenden Erfindung ein z.B. nach den bekannten Methoden des Standes der Technik hergestellter Formkörper, z.B. eine Faser oder ein Bändchen, verstanden.

Im Gegensatz zum Stand der Technik gefalteter PTFE-Produkte wird bei der vorliegenden Erfindung der Vorformkörper nicht gefaltet, sondern vollständig verdreht bzw. verzwirnt.

Dabei erweist es sich als vorteilhaft, wenn der Vorformkörper mit 30 Drehungen pro m (T/m) bis 400 Drehungen pro m (T/m) verdreht wird.

Der Vorformkörper kann vorzugsweise um sich selbst verdreht werden. In diesem Fall ist es günstig, wenn der Vorformkörper mit 220 Drehungen pro m bis 270 Drehungen pro m, besonders bevorzugt 245 Drehungen pro m bis 255 Drehungen pro m verdreht wird. Im Falle eines im wesentlichen aus PTFE bestehenden Vorformkörpers resultiert dabei ein im wesentlichen aus PTFE bestehender Formkörper.

Eine bevorzugte Ausführungsform des erfindungsgemäßen Verfahrens ist dadurch gekennzeichnet, daß der Vorformkörper aus Polytetrafluorethylen um ein anderes Material herum verdreht wird. Dieses andere Material kann z.B. ein Mono- oder Multifilament, bevorzugt aus Polyamid, Polyester oder wiederum Polytetrafluorethylen sein. Das andere Material kann jedoch auch ein Spinngarn, bevorzugt aus Baumwolle, Viscose, Polyamid, Polyester oder Acryl sein.

Durch diese Ausführungsform des erfindungsgemäßen Verfahrens wird es möglich, die Eigenschaften des umdrehten anderen Materials, z.B. höhere Festigkeit, mit den vorteilhaften Eigenschaften von PTFE, insbesondere der Geschmeidigkeit und der guten Gleiteigenschaft zu verbinden.

Der Titer des anderen Materials liegt vorzugsweise im Bereich zwischen 100 dtex und 1000 dtex, besonders bevorzugt zwischen 300 dtex und 500 dtex.

Wenn der Vorformkörper aus PTFE um ein anderes Material herum verdreht wird, ist es vorteilhaft, daß der Vorformkörper mit 50 Drehungen pro m bis 250 Drehungen pro m, vorzugsweise 100 Drehungen pro m bis 150 Drehungen pro m, um das andere Material herum verdreht wird.

Eine weitere vorteilhafte Ausführungsform des erfindungsgemäßen Verfahrens besteht darin, daß das andere Material Geschmacksstoffe und/oder der Zahnhygiene dienende Stoffe enthält.

Es ist bekannt, daß PTFE-Formkörper, insbesondere wenn sie als Zahnseide verwendet werden, Geschmacksstoffe und/oder der Zahnhygiene dienende Stoffe enthalten. Dafür verwendbare Stoffe sind z.B. in der PCT-WO 95/34252 beschrieben.

Es erweist sich als günstig, wenn solche Zusatzstoffe im anderen Material, um welches der Vorformkörper herum gedreht wird, enthalten sind. Diese Zusatzstoffe können z.B. als Depot dienen.

Vorzugsweise wird der verdrehte Vorformkörper durch Kalandrieren zwischen vorzugsweise 2 Walzen flachgedrückt. Insbesondere bei dieser Verfahrensweise ist es möglich, das erfindungsgemäße Verfahren kontinuierlich durchzuführen.

Der eingesetzte Vorformkörper ist bevorzugt ein monoaxial verstreckter Formkörper aus PTFE. Der Vorformkörper kann vor dem Verdrehen bevorzugt bändchenförmig sein. Der Titer des Vorformkörpers kann zwischen 400 dtex bis 1800 dtex, bevorzugt 600 dtex bis 1200 dtex, liegen.

Besonders gut für das erfindungsgemäße Verfahren eignen sich monoaxial verstreckte Vorformkörper, welche einen Festigkeitswert in Verstreckungsrichtung von mindestens 20 cN/tex, vorzugsweise 25 cN/tex bis 30 cN/tex und eine Dichte von weniger als 2 g/cm³, vorzugsweise von weniger als 1,8 g/cm³, besonders bevorzugt von 1,6 g/cm³ bis 1,8 g/cm³ aufweisen. Solche Vorformkörper können z.B. ähnlich den in der EP 0 391 887 A1 oder der US-A 3,953,566 beschriebenen Verfahren hergestellt werden.

Es erweist sich weiters als günstig, wenn der Vorformkörper Geschmacksstoffe und/oder der Zahnhygiene dienende Stoffe enthält.

Insbesondere für die Verwendung als Zahnseide ist es vorteilhaft, wenn auf den durch das erfindungsgemäße Verfahren erhaltenen Formkörper eine Beschichtung aus Wachs aufgebracht wird. Auch das auf den Formkörper aufgebrachte Wachs kann Geschmacksstoffe und/oder der Zahnhygiene dienende Stoffe enthalten.

Die Aufgabe der vorliegenden Erfindung wird auch durch einen fibrillationsarmen Formkörper enthaltend Polytetrafluorethylen gelöst, welcher durch das erfindungsgemäße Verfahren erhältlich ist.

Die erfindungsgemäßen Formkörper erweisen sich als besonders widerstandsfähig gegen Fibrillierung und weisen in dieser Beziehung bessere Eigenschaften als Formkörper des Standes der Technik auf. Durch die Drehung werden die Oberfläche und speziell die Kanten des Formkörpers etwas rauher. Dies ergibt insbesondere bei der Verwendung des erfindungsgemäßen Formkörpers als Zahnseide einen besseren Reinigungseffekt als bei bekannten, ungedrehten Materialien.

Die erfindungsgemäßen Formkörper eignen sich somit in hervorragender Weise insbesondere als Material für Zahnseide, wo durch die günstigen Eigenschaften des Materials die vom Verwender einer Zahnseide gewünschten Merkmale, nämlich einerseits Geschmeidigkeit und andererseits Festigkeit und Widerstandsfähigkeit gegen Fibrillierung, verbunden mit einer guten Reinigungswirkung besonders wirkungsvoll erfüllt werden.

### Beispiele:

### Beispiel 1 - Verdrehtes und kalandriertes PTFE-Bändchen:

Ein PTFE-Bändchen mit 2,2 mm Breite, 35 pm Dicke, einer Festigkeit von 27 cN/tex, 7 % Dehnung und einer Dichte von 1,8 g/cm³ wurde mit 250 Umdrehungen pro m (T/m) verdreht und anschließend zwischen 2 Walzen wiederum zur Bändchenform flach gedrückt. Man erhält ein Bändchen mit 50 µm Dicke, 1,3 mm Breite und 1,9 g/cm³ Dichte. Die Festigkeit beträgt 25 cN/tex bei 10 % Dehnung.

### Beispiel 2a: Verdrehung um ein Polyestermultifilamentgarn:

Ein Polyestermultifilamentgarn (320 dtex) wird mit einem PTFE-Bändchen von 800 dtex mit 100 T/m umsponnen. Dies geschieht auf einer Ringzwirnmaschine durch unterschiedliche Einstellung der Fadenspannung der zugeführten Materialien (PET-Garn 100 g, PTFE-Bändchen 20 g).

Das erhaltene mit einem PTFE-Bändchen umdrehte Garn wird wie im Beispiel 1 beschrieben flachgedrückt. Man erhält ein Bändchen mit 1,5 mm Breite und 50 µm Dicke. Die Reißkraft beträgt 35 N bei einer Dehnung von 15 %.

### Beispiel 2b: Verdrehung um ein Polyamidmultifilamentgarn:

Wie Beispiel 2a, jedoch unter Verwendung eines Polyamidmultifilamentgarnes von 480 dtex, welches mit dem PTFE-Bändchen umsponnen wurde.

Das erhaltene Bändchen hat einen Gesamttiter von 1300 dtex bei einer Breite von 1,5 mm und 55 µm Dicke. Dieses Bändchen wird mit 10 % Bienenwachs präpariert.

### Beispiel 2c: Verdrehung um ein Spinngarn:

Ein Baumwollspinngarn mit 500 dtex wird mit einem PTFE-Bändchen von 800 dtex gemäß Beispiel 2a umsponnen und anschließend flachgedrückt.

Man erhält ein Bändchen mit 1,5 mm Breite, 60 pm Dicke und 30 N Reißkraft. Dieses Bändchen wird mit 8 % Bienenwachs, welches 1 % Pfefferminzöl enthält, präpariert.

### Beispiel 3a: Verwendung von Zusatzstoffen und/oder der Zahnhygiene dienenden Stoffen:

Es wird wie im Beispiel 2a verfahren, jedoch wird das Polyester-Multifilamentgarn vor dem Umdrehen mit dem PTFE-Bändchen mit 3% Desinfektionsmittel getränkt.

### Beispiel 3b:

Es wird wie im Beispiel 2c verfahren, jedoch wird das Baumwollgarn vor dem Umdrehen mit dem PTFE-Bändchen mit Citrusgeschmack versetzt.

## Patentansprüche

1. Verfahren zur Herstellung eines fibrillationsarmen Formkörpers enthaltend Polytetrafluorethylen, dadurch gekennzeichnet, daß ein Vorformkörper aus Polytetrafluorethylen verdreht und anschließend wiederum flachgedrückt wird.

2. Verfahren gemäß Anspruch 1, dadurch gekennzeichnet, daß der Vorformkörper mit 30 Drehungen pro m bis 400 Drehungen pro m verdreht wird.

3. Verfahren nach Anspruch 1 oder 2, dadurch gekennzeichnet, daß der Vorformkörper um sich herum verdreht wird.

4. Verfahren gemäß Anspruch 3, dadurch gekennzeichnet, daß der Vorformkörper mit 220 Drehungen pro m bis 270 Drehungen pro m, besonders bevorzugt 245 Drehungen pro m bis 255 Drehungen pro m verdreht wird.

5. Verfahren gemäß Anspruch 1 oder 2, dadurch gekennzeichnet, daß der Vorformkörper aus Polytetrafluorethylen um ein anderes Material herum verdreht wird.

6. Verfahren gemäß Anspruch 5, dadurch gekennzeichnet, daß das andere Material ein Mono- oder Multifilament, bevorzugt aus Polyamid, Polyester oder Polytetrafluorethylen ist.

7. Verfahren gemäß Anspruch 5, dadurch gekennzeichnet, daß das andere Material ein Spinngarn, bevorzugt aus Baumwolle, Viscose, Polyamid, Polyester oder Acryl ist.

8. Verfahren gemäß einem der Ansprüche 5 bis 7, dadurch gekennzeichnet, daß der Titer des anderen Materials im Bereich zwischen 100 dtex und 1000 dtex, vorzugsweise zwischen 300 dtex und 500 dtex liegt.

9. Verfahren gemäß einem der Ansprüche 5 bis 8, dadurch gekennzeichnet, daß der Vorformkörper mit 50 Drehungen pro m bis 250 Drehungen pro m, vorzugsweise 100 Drehungen pro m bis 150 Drehungen pro m um das andere Material herum verdreht wird.

10. Verfahren gemäß einem der Ansprüche 5 bis 9, dadurch gekennzeichnet, daß das andere Material Geschmackstoffe und/oder der Zahnhygiene dienende Stoffe enthält.

11. Verfahren gemäß einem der vorhergehenden Ansprüche, dadurch gekennzeichnet, daß der verdrehte Vorformkörper durch Kalandrieren zwischen vorzugsweise 2 Walzen flachgedrückt wird.

12. Verfahren gemäß einem der vorhergehenden Ansprüche, dadurch gekennzeichnet, daß der Vorformkörper monoaxial verstreckt ist.

13. Verfahren gemäß einem der vorhergehenden Ansprüche, dadurch gekennzeichnet, daß der Vorformkörper vor dem Verdrehen bändchenförmig ist.

14. Verfahren gemäß einem der vorhergehenden Ansprüche, dadurch gekennzeichnet, daß der Titer des Vorformkörpers vor dem Verdrehen 400 dtex bis 1800 dtex, bevorzugt 600 dtex bis 1200 dtex ist.

15. Verfahren gemäß einem der vorhergehenden Ansprüche, dadurch gekennzeichnet, daß der monoaxial verstreckte Vorformkörper einen Festigkeitswert in Verstreckungsrichtung von mindestens 20 cN/tex, vorzugsweise 25 cN/tex bis 30 cN/tex und eine Dichte von weniger als 2 g/cm³, vorzugsweise von weniger als 1,8 g/cm³, besonders bevorzugt von 1,6 g/cm³ bis 1,8 g/cm³ aufweist.

16. Verfahren gemäß einem der vorhergehenden Ansprüche, dadurch gekennzeichnet, daß der Vorformkörper Geschmackstoffe und/oder der Zahnhygiene dienende Stoffe enthält.

17. Verfahren gemäß einem der vorhergehenden Ansprüche, dadurch gekennzeichnet, daß auf den erhaltenen Formkörper eine Beschichtung aus Wachs aufgebracht wird.

18. Verfahren gemäß Anspruch 17, dadurch gekennzeichnet, daß das Wachs Geschmackstoffe und /oder der Zahnhygiene dienende Stoffe enthält.

19. Fibrillationsarmer Formkörper enthaltend Polytetrafluorethylen, erhältlich durch ein Verfahren gemäß einem der Ansprüche 1 bis 18.

20. Verwendung eines fibrillationsarmen Formkörpers enthaltend Polytetrafluorethylen gemäß Anspruch 19 als Zahnseide material.

## Claims

1. Process for the production of a low-fibrillation moulded body containing polytetrafluorethylene, characterised in that a pre moulded body of polytetrafluorethylene is twisted and then pressed flat.

2. Process according to claim 1 characterised in that the pre moulded body is twisted with 30 twists per m up to 400 twists per m.

3. Process according to claim 1 or 2, characterised in that the pre moulded body is twisted around itself.

4. Process according to claim 3, characterised in that the pre moulded body is twisted with 220 twists per m up to 270 twists per m, and preferably 245 twists per m up to 255 twists per m.

5. Process according to claim 1 or 2, characterised in that the pre moulded body of polytetrafluorethylene is twisted around another material.

6. Process according to claim 5, characterised in that the other material is a mono or multifilament, preferably of polyamide, polyester or polytetrafluorethylene.

7. Process according to claim 5, characterised in that the other material is a spun yarn, perferably of cotton, viscose, polyamide, polyester or acrylic.

8. Process according to one of claims 5 to 7, characterised in that the titre of the other material lies in the range of between 100 dtex and 1000 dtex, preferably between 300 dtex and 500 dtex.

9. Process according to one of claims 5 to 8, characterised in that the pre moulded body is twisted with 50 twists per m up to 250 twists per m, preferably 100 twists per m up to 150 twists per m around the other material.

10. Process according to one of claims 5 to 9, characterised in that the other material contains flavourings and / or substances which serve dental hygiene.

11. Process according to one of the preceding claims characterised in that the twisted pre moulded body is pressed flat by calendering preferably between 2 rolls.

12. Process according to one of the preceding claims characterised in that the pre moulded body is monoaxially stretched.

13. Process according to one of the preceding claims characterised in that the pre moulded body is tape-like prior to twisting.

14. Process according to one of the preceding claims charactersied in that the titre of the pre moulded body is 400 dtex to 1800 dtex prior to twisting, and preferably 600 dtex to 1200 dtex.

15. Process according to one of the preceding claims characterised in that the monoaxially stretched pre moulded body has a tenacity value in the stretching direction of at least 20 cN/tex, and preferably 25 cN/tex to 30 cN/tex and a density of less than 2 g/cm³, preferably of less than 1.8 g/cm³, especially preferred of 1.6 g/cm³ to 1.8 g/cm³.

16. Process according to one of the preceding claims characterised in that the pre moulded body contains flavourings and / or substances which serve dental hygiene.

17. Process according to one of the preceding claims characterised in that a coating of wax is applied to the moulded body obtained.

18. Process according to claim 17, characterised in that the wax contains flavourings and / or substances which serve dental hygiene.

19. Low-fibrillation moulded body containing polytetrafluorethylene, obtainable by means of a process in accordance with one of claims 1 to 18.

20. Use of a low-fibrillation moulded body containing polytetrafluorethylene in accordance with claim 19 as dental floss material.

## Revendications

1. Procédé de fabrication d'un corps façonné faiblement fibrillé, contenant du polytétrafluoréthylène, caractérisé par le fait qu'une préforme du corps en polytétrafluoréthylène est tordue et ensuite comprimée de nouveau à plat.

2. Procédé selon la revendication 1, caractérisé par le fait que la préforme est tordue avec 30 à 400 torsions par m.

3. Procédé selon la revendication 1 ou 2, caractérisé par le fait que la préforme est tordue autour d'elle-même.

4. Procédé selon la revendication 3, caractérisé par le fait que la préforme est tordue avec 220 torsions à 270 torsions par m, mais de préférence avec 245 torsions à 255 torsions par m.

5. Procédé selon la revendication 1 ou 2, caractérisé par le fait que la préforme en polytétrafluoréthylène est tordue autour d'un autre matériau.

6. Procédé selon la revendication 5, caractérisé par le fait que l'autre matériau est un monofilament ou un multifilament, de préférence en polyamide, polyester ou polytétrafluoréthylène.

7. Procédé selon la revendication 5, caractérisé par le fait que l'autre matériau est un fil de filature, de préférence en coton, viscose, polyamide, polyester ou acryl.

8. Procédé selon l'une des revendications 5 à 7, caractérisé par le fait que le titre de l'autre matériau se trouve dans la plage de 100 dtex à 1000 dtex, de préférence de 300 dtex à 500 dtex.

9. Procédé selon l'une des revendications 5 à 8, caractérisé par le fait que la préforme est tordue autour de l'autre matériau avec 50 torsions à 250 torsions par m, de préférence avec 100 torsions à 150 torsions par m.

10. Procédé selon l'une des revendications 5 à 9, caractérisé par le fait que l'autre matériau contient des aromates voire des substances servant à l'hygiène dentaire.

11. Procédé selon l'une des revendications précédentes, caractérisé par le fait que la préforme tordue est comprimée à plat par des calandres entre de préférence 2 cylindres.

12. Procédé selon l'une des revendications précédentes, caractérisé par le fait que la préforme est étirée de manière monoaxiale.

13. Procédé selon l'une des revendications précédentes, caractérisé par le fait que la préforme est sous forme de bandelettes avant la torsion.

14. Procédé selon l'une des revendications précédentes, caractérisé par le fait que le titre de 1 la préforme est avant la torsion de 400 dtex à 1800 dtex, de préférence de 600 dtex à 1200 dtex.

15. Procédé selon l'une des revendications précédentes, caractérisé par le fait que la préforme étirée de manière monoaxiale a une valeur de résistance dans la direction d'étirage d'au moins 20 cN/tex, de préférence de 25 cN/tex à 30 cN/tex et une densité de moins de 2 g/cm³, de préférence de moins de 1,8 g/cm³, et encore mieux de 1,6 g/cm³ à 1,8 g/cm³.

16. Procédé selon l'une des revendications précédentes, caractérisé par le fait que la préforme contient des aromates voire des substances servant à l'hygiène dentaire.

17. Procédé selon l'une des revendications précédentes, caractérisé par le fait qu'une couche de cire est appliquée sur la préforme obtenue.

18. Procédé selon la revendication 17, caractérisé par le fait que la cire contient des aromates voire des substances servant à l'hygiène dentaire.

19. Corps façonné faiblement fibrillé, contenant du polytétrafluoréthylène, pouvant être obtenu par un procédé selon l'une des revendications 1 à 18.

20. Utilisation d'une préforme faiblement fibrillée, contenant du polytétrafluoréthylène selon la revendication 19 en tant que soie dentaire.
